# EUROPÄISCHE PATENTANMELDUNG

(11) **EP 1 946 698 A2**
(43) Veröffentlichungstag der Anmeldung: **23.07.2008**
(21) Anmeldenummer: 08100367.5
(22) Anmeldetag: 11.01.2008
(51) Int. Cl.: A61B 5/0404, A61B 5/0408, A61B 5/11

(54) **Medizinische Trageeinrichtung**

(30) Priorität: 13.01.2007 DE 102007002058
(71) Anmelder: Enverdis GmbH, 07745 Jena (DE)
(72) Erfinder: Hübner, Thomas, 07749, Jena (DE); Alt, Michael, 36275, Kirchheim (DE)
(74) Vertreter: von Kirschbaum, Alexander

(57) **Zusammenfassung**

Eine medizinische Trageeinrichtung zum körpernahen Tragen medizinischer Messeinrichtungen weist ein Gurtsystem (10, 12, 14, 16, 22, 26, 28) mit einer Schließeinrichtung (18) zum Öffnen und Schließen auf. Mit dem Gurtsystem sind Halteeinrichtungen (30) zur Aufnahme von EKG-Elektroden verbunden. Ferner ist mit dem Gurtsystem eine Aufzeichnungseinrichtung (40) zur Aufzeichnung von Messsignalen verbunden.

## Beschreibung

Die Erfindung betrifft eine medizinische Trageeinrichtung zum körpernahen Tragen medizinischer Messeinrichtungen.

Zur Durchführung eines EKGs werden Elektroden an definierten Stellen des menschlichen Körpers befestigt. Üblicherweise werden mehrere Elektroden im Bereich des Brustkorbs, im Bereich der Schlüsselbeine und ggf. dem Rücken befestigt. Hierbei werden häufig Klebeelektroden verwendet, die unmittelbar auf die Haut des Patienten geklebt werden. Jede einzelne Elektrode ist hierbei über Leitungen mit einer Auswerteeinrichtung verbunden. Neben herkömmlichen Ruhe-EKGs werden auch Belastungs-EKGs durchgeführt. Bei Belastungs-EKGs wird der Patient durch eine körperliche Belastung, wie beispielsweise Radfahren, belastet. Aus medizinischen Gründen ist es bei der Durchführung von Belastungs-EKGs erforderlich, dass ein Arzt anwesend ist. Ferner muss ein Defibrillator zur eventuellen Wiederbelebung des Patienten vorhanden sein. Anstelle oder zusätzlich zu Belastungs-EKGs sind Langzeit-EKGs zur Diagnose hilfreich, bei denen ein EKG während herkömmlicher Alltagsbelastung aufgenommen wird. Hierbei besteht die Problematik, dass die einzelnen Elektroden verrutschen oder sich von der Körperoberfläche lösen.

US 5,445,149 beschreibt eine Tragevorrichtung zum Tragen von EKG-Elektroden am menschlichen Oberkörper. Die Tragevorrichtung umfasst einen Brustbereich sowie einen Lendenbereich, in denen jeweils Elektroden angeordnet sind. Ferner sind Schultergurte vorgesehen, in denen weitere Elektroden befestigt sind. Zur Anpassung an verschiedene körperliche Gegebenheiten von Patienten ist es vorgesehen, Trageeinrichtungen mit verschiedenen Größen zu verwenden. Auf diese Weise können die EKG-Elektroden bei einer Vielzahl von Patienten korrekt positioniert werden.

Aufgabe der Erfindung ist es, eine medizinische Trageeinrichtung zu schaffen, mit der das Durchführen von EKGs, insbesondere von Belastungs- und Langzeit-EKGs vereinfacht wird.

Die Lösung der Aufgabe erfolgt erfindungsgemäß durch die Merkmale des Anspruchs 1.

Die erfindungsgemäße Trageeinrichtung weist ein Gurtsystem auf, das vom Patienten körpernah getragen werden kann. Insbesondere wird das Gurtsystem am Oberkörper des Patienten angelegt. Hierzu weist das Gurtsystem eine Schließeinrichtung zum Öffnen und Schließen des Gurtsystems auf, um ein einfaches Anlegen des Gurtsystems am Körper des Patienten zu ermöglichen. Erfindungsgemäß weist das Gurtsystem mindestens zwei, vorzugsweise mehrere Halteeinrichtungen auf. Die mindestens zwei Halteeinrichtungen dienen zur Aufnahme jeweils einer EKG-Elektrode, Hierbei kann die Elektrode auswechselbar mit der Halteeinrichtung verbunden sein. Hierbei ist die Halteeinrichtung vorzugsweise derart mit dem Gurtsystem verbunden, dass die Elektrode zwischen dem Gurtsystem und der Hautoberfläche des Patienten angeordnet ist. Die Elektrode wird somit durch das Gurtsystem, das vorzugsweise elastisch ausgebildet ist, oder zumindest elastisch ausgebildete Bereiche aufweist, an den Körper gedrückt. Hierdurch ist der Patient in seiner Bewegungsfreiheit nicht oder nur geringfügig eingeschränkt. Ferner ist sichergestellt, dass auch beim Bewegen des Patienten die Elektrode nicht verrutscht oder sich gar von der Hautoberfläche löst. Ferner ist erfindungsgemäß mit dem Gurtsystem eine Aufzeichnungseinrichtung verbunden. Durch die Aufzeichnungseinrichtung können die von den Elektroden abgegebenen Messsignale aufgezeichnet und vorzugsweise zwischengespeichert werden.

Beim Vorsehen von beispielsweise zwei Elektroden ist es möglich, ein einkanaliges EKG abzuleiten. Je nach Anforderungen an das EKG können bis zu drei oder fünf Elektroden oder ggf. auch mehr Elektroden vorgesehen sein. Die Elektroden sind jeweils an anatomisch definierten Fixpunkten anzuordnen. Dies ist mit Hilfe des erfindungsgemäßen Gurtsystems gut möglich, wobei die Halteeinrichtungen hierbei vorzugsweise mit einem Justageelement zum Einstellen der Lageelektroden verbunden sind. Insbesondere weist jede Halteeinrichtung ein derartiges Justageelement auf. Vorzugsweise ist jede Elektrode mit einer gesonderten Halteeinrichtung verbunden und die Lage der entsprechenden Elektrode durch die Justageeinrichtung exakt definierbar, Bei einer Ableitung von fünf Elektroden werden diese Elektroden vorzugsweise folgendermaßen angeordnet:
1, Manubrium sterni
2. Processus xiphoideus
3. vorderen Axillarlinie links in Höhe des 5. ICR
4. vorderen Axillarlinie rechts in Höhe des 5. ICR
5. eine Referenzelektrode, so herzfern wie möglich

Beim Vorsehen von drei Elektroden werden diese vorzugsweise an den folgenden anatomischen Punkten des Patienten angeordnet:
1. unterhalb der rechten Clavicula
2. Processus xiphoideus
3. unterhalb der linken Clavicula

Die erfindungsgemäße medizinische Trageeinrichtung kann somit vom Patienten im Alltag getragen werden, wobei durch das Vorsehen der mit dem Gurtsystem verbundenen Aufzeichnungseinrichtung über einen langen Zeitraum Daten aufgezeichnet werden können,

Vorzugsweise ist das Aufzeichnungssystem unmittelbar mit dem Gurtsystem verbunden. Es ist jedoch auch möglich, dass die Aufzeichnungseinrichtung beispielsweise am Hosengürtel befestigt wird oder in einer gesonderten Tragetasche angeordnet ist. Hierbei ist die Aufzeichnungseinrichtung über elektrische Leitungen oder drahtlos mit den Elektroden verbunden. Besonders bevorzugt ist es hierbei, dass die Aufzeichnungseinrichtung über elektrische Leitungen mit Elektroden verbunden ist und an dem Gurtsystem befestigt ist. Hierdurch können relativ kurze elektrische Leitungen vorgesehen werden, so dass eine Beeinträchtigung des Patienten durch Leitungen vermieden ist. Ferner wird hierdurch vermieden, dass durch die Bewegung des Patienten Leitungen beschädigt werden oder Leitungen aus Kontakten herausrutschen, Besonders bevorzugt ist es hierbei, dass die Leitungen die zwischen der Aufzeichnungseinrichtung und den Halteeinrichtungen bzw. Elektroden angeordnet sind, fest mit dem Gurtsystem verbunden sind. Insbesondere ist es vorteilhaft, dass die elektrischen Leitungen in das Gurtsystem integriert, d. h. innerhalb der Gurte angeordnet sind. Da es sich bei dem Gurtsystem insbesondere um aus Kunststoffmaterial hergestellte Gurte handelt, ist es möglich, die elektrischen Leitungen in das Kunststoffmaterial einzugießen. Unabhängig hiervon kann an der Innenseite des insbesondere aus einem elastischem Kunststoff hergestellten Gurtsystems eine hautfreundliche Auflage vorgesehen sein.

Das Gurtsystem umfasst vorzugsweise mindestens einen Brustgurt. Der Brustgurt kann um den Brustkorb des Patienten angeordnet werden, so dass ein Vorderteil des Brustgurtes an der Vorderseite des Körpers und ein Rückenteil des Brustgurtes an dem Rücken des Patienten anliegt. Um den Tragkomfort zu erhöhen und auch Brustkorbbewegungen zuzulassen, ist der Brustgurt aus elastischem Material ausgebildet und/oder weist ein elastisches Gurtteil auf, bei dem es sich beispielsweise um ein elastisches Band handeln kann, das insbesondere zwischen dem Vorderteil und dem Rückenteil des Brustgurtes angeordnet ist.

In einer besonders bevorzugten Ausführungsform weist das Gurtsystem neben dem Brustgurt mindestens einen, vorzugsweise zwei Schultergurte auf. Die Schultergurte sind vorzugsweise mit dem Vorderteil, sowie dem Rückenteil des Brustgurtes verbunden. Vorzugsweise erfolgt das Verbinden der Schultergurte mit dem Vorderteil durch einen Brustbeingurt. Der Brustbeingurt ist im Bereich des Brustbeins angeordnet, wenn die medizinische Trageeinrichtung vom Patienten getragen wird. Vorzugsweise verläuft der Brustbeingurt bis zum Ansatz der Schlüsselbeine, wobei die Schultergurte sodann entlang der Schlüsselbeine über die Schulter des Patienten nach hinten geführt sind. Der Brustbeingurt ist somit in Verbindung mit den Schultergurten an der Vorderseite des Oberkörpers des Patienten Y-förmig ausgebildet. Insbesondere ist der Brustbeingurt mit den beiden Schultergurten einstückig ausgebildet. Die Verbindung der beiden Schultergurte mit dem Rückenteil des Brustgurtes erfolgt beispielsweise an zwei zueinander beabstandeten Verbindungsstellen. Hierbei sind die Verbindungsstellen vorzugsweise derart gewählt, dass die Schultergurte am Rücken des Patienten im Wesentlichen vertikal verlaufen. Hierdurch ist gewährleistet, dass die Schultergurte im Bereich der Schlüsselbeine anliegen und nicht verrutschen. Ferner ist der Tragekomfort hierdurch erhöht. Ebenso können die beiden Schultergurte zu einem Rückengurt, der insbesondere im Bereich der Wirbelsäule verläuft, zusammengefasst werden.

Die Schließeinrichtung des Gurtsystems, die vorzugsweise im Bereich des Thorax angeordnet ist, ist vorzugsweise mit dem Brustgurt und dem Brustbeingurt verbunden. Hierbei kann sowohl eine Verbindung der Schließvorrichtung mit dem Brustgurt als auch die Verbindung mit dem Brustbeingurt lösbar sein. Ausreichend ist es, eine lösbare Verbindung des Brustgurtes vorzusehen, um ein einfaches Anlegen der Trageeinrichtung zu ermöglichen. Da die elektrischen Leitungen vorzugsweise fest mit dem Gurtsystem verbunden und insbesondere in das Gurtsystem integriert sind, kann die Schließeinrichtung derart ausgebildet sein, dass elektrische Kontakte vorgesehen sind. Vorzugsweise sind die elektrischen Kontakte in die Schließeinrichtung integriert, so dass die elektrischen Kontakte beim Schließen der Schließeinrichtung automatisch verbunden werden. In bevorzugter Ausführungsform ist die Aufzeichnungseinrichtung über ein Kabel mit der Schließeinrichtung verbunden. Hierdurch können die elektrischen Kontakte in der Schließeinrichtung ggf. entfallen.

Um die Lage der EKG- Elektroden individuell an jeden Patienten anpassen zu können, weisen die mindestens zwei Halteeinrichtungen vorzugsweise ein Justageelement auf. Hierbei ist das Justageelement vorzugsweise derart ausgebildet, dass ein Verschieben der Elektrode zumindest in Längsrichtung des Gurts möglich ist. Vorzugsweise weist das Justageelement hierzu eine schlitzförmige Öffnung auf, in der ein stiftförmiger Ansatz verschiebbar gehalten ist. Der Schlitz ist hierbei in bevorzugter Ausführungsform in einem Winkel zur Längsrichtung des Gurtes angeordnet, um eine gute Justage der Elektrode zu ermöglichen. Ggf. ist das Justagelement so ausgebildet, dass die Orientierung der schlitzförmigen Öffnung durch Drehen des Justageelements bzw. der gesamten Halteeinrichtung verändert werden kann. Vorzugsweise ist der stiftförmige Ansatz hierbei unmittelbar mit der Elektrode oder einem Aufnahmeelement, das die Elektrode hält, verbunden. Die schlitzförmige Öffnung ist sodann in einem mit dem Gurt verbundenen Element vorgesehen. Selbstverständlich kann auch der stiftförmige Ansatz mit dem Gurt verbunden sein, wobei sodann die schlitzförmige Öffnung mit dem Aufnahmeelement für die Elektrode verbunden ist. Hierbei kann die Lage der Elektrode vorzugsweise derart verändert werden, dass der Mittelpunkt der Elektrode innerhalb eines Kreises von 1 bis 2 cm durch Drehen des Justagelements und Verschieben in dem Schlitz angeordnet werden kann.

Vorzugsweise ist die Halteeinrichtung, insbesondere der Stift, derart ausgebildet, dass ein Verbinden mit einer handelsüblichen Einweg-Druckknopfelektrode möglich ist. Geeignet sind beispielsweise handelsübliche Elektroden, bei denen es sich insbesondere um Einmal-Druckknopf-Feuchtelektroden oder wiederverwendbare Druckknopf-Trockenelektroden handelt. Hierbei kann es sich grundsätzlich um Trocken- oder Feuchtelektroden handeln. Die mit der Halteeinrichtung bzw. dem stiftförmigen Ansatz verbundene Druckknopf-Aufnahme ist dementsprechend handelsüblich ausgebildet.

Vorzugsweise weist die medizinische Trageeinrichtung eine Überwachungseinrichtung zur Überwachung der Funktionsfähigkeit der Elektroden auf. Diese Überwachungseinrichtung kann in die Aufzeichnungseinrichtung integriert sein. Insbesondere weist die Überwachungseinrichtung zusätzlich Anzeigemittel wie LEDs auf, die insbesondere an der Schließeinrichtung angeordnet sein können. Beispielsweise über eine Widerstandsmessung kann überprüft werden, ob die angelegten Elektroden Signale mit ausreichender Qualität erzeugen.

Insbesondere bei Langzeit-EKGs ist es erforderlich, dass der Patient jede körperliche Belastung, wie beispielsweise das Treppensteigen unter Angabe der entsprechenden Zeit notiert. Dies ist äußerst umständlich und häufig auch ungenau, da vom Patienten häufig nicht sämtliche Belastungen notiert werden. Vorzugsweise ist die erfindungsgemäße medizinische Trageeinrichtung daher mit einer Aufnahmeeinrichtung zur Aufnahme von Audiosignalen verbunden. Die Aufnahmeeinrichtung weist hierzu ein Mikrophon auf, das mit dem Gurtsystem, insbesondere dem Schultergurt, verbunden ist. Die von dem Mikrophon aufgenommenen Audiosignale werden aufgezeichnet. Dies erfolgt vorzugsweise durch die bereits zur Aufzeichnung der EKG-Signale vorhandene Aufzeichnungseinrichtung. Hierzu ist das Mikrophon über elektrische Leitungen mit der Aufzeichnungseinrichtung verbunden. Die Aufzeichnung der von der Aufnahmeeinrichtung aufgenommenen Audiosignale mit Hilfe der vorhandenen Aufzeichnungseinrichtung hat insbesondere den Vorteil, dass die Audiosignale unmittelbar den EKG-Signalen zugeordnet werden können. Dies kann beispielsweise durch eine entsprechende, insbesondere durch Software erfolgende Verknüpfung geschehen. Auch kann zusätzlich ein Zeitsignal, bei dem es sich beispielsweise um die Uhrzeit handeln kann, aufgezeichnet werden. Durch das erfindungsgemäße Vorsehen einer Aufnahmeeinrichtung muss der Patient bei einem Langzelt-EKG die von ihm durchgeführten Tätigkeiten nicht mehr schriftlich notieren. Vielmehr ist es ausreichend, dass der Patient beispielsweise durch Drücken eines Knopfes die Aufnahmeeinrichtung aktiviert, und die entsprechende Tätigkeit in das Mikrophon spricht. Ein entsprechender Aktivierungsknopf kann beispielsweise neben dem Mikrophon am Schultergurt vorgesehen sein.

Eine unabhängige Erfindung betrifft ferner ein Verfahren zur Erfassung eines Langzeit-EKGs, wobei insbesondere eine Vorrichtung, wie sie in der vorliegenden Anmeldung beschrieben ist, verwendet werden kann. Erfindungsgemäß werden EKG-Signale durch eine Aufzeichnungseinrichtung aufgezeichnet. Zeitgleich werden Audiosignale durch eine Aufnahmevorrichtung aufgenommen. Es erfolgt ein automatisiertes Zuordnen der EKG-Signale zu den entsprechenden Audiosignalen.

Anstelle der Aufnahme von Audiosignalen oder zusätzlich zu einer solchen Aufnahme, kann auch die Beschleunigung von Körperteilen eines Patienten durch einen Beschleunigungssensor und/oder die Bewegung von Körperteilen eines Patienten durch einen Positionserfassungssensor erfasst werden.

Vorzugsweise weist die medizinische Trageeinrichtung eine mit dem Gurtsystem verbundene Zusatz-Halteeinrichtung zur Verbindung mit medizinischen Sensoren oder Modulen auf, die während einer Langzeitaufzeichnung vom Patienten mitgetragen werden können. Über die Zusatz-Halteeinrichtung kann beispielsweise ein konventionelles, mit Manschettendruck nach auskultatorischem und/oder oszillometrischem Messprinzip arbeitendem, Blutdruckmodul verbunden werden. Die von dem Blutdruckmessgerät oder einem anderen medizinischen Messgerät gemessenen Daten werden hierbei vorzugsweise wiederum an die Aufzeichnungseinrichtung übermittelt. Hierzu ist vorzugsweise auch die Zusatz-Halteeinrichtung mit elektrischen Kontakten versehen. Über die elektrischen Kontakte kann eine elektrische Verbindung zu dem medizinischen Messgerät erfolgen. Über elektrische Leitungen, die zwischen den Kontakten und der Aufzeichnungseinrichtung vorgesehen, insbesondere in das Gurtsystem integriert sind, erfolgt sodann die Übertragung der Messdaten.

Zur Blutdruckermittlung kann am Gurtsystem beispielsweise ein fotoplethysmografischer Sensor zur kontinuierlichen Blutdruckermittlung vorgesehen sein. Dieser wird vorzugsweise am Ohrläppchen angeordnet. Die von dem Sensor ausgehenden Kabel werden vorzugsweise mit dem Schultergurt des Gurtsystems verbunden bzw. sind in diese integriert. Derartige Sensoren arbeiten üblicherweise kontinuierlich mit mindestens einer Wellenlänge, Vorzugsweise sind mehrere Wellenlängen im Bereich von 400 nm bis 1650 nm als Transmissions- und/oder Reflektionssensoren vorgesehen. Ferner ist es auch möglich, ein herkömmliches Blutdruckmessmodul, wie eine Armmanschette, mit dem Gurtsystem zu verbinden.

Mit der mindestens einen Zusatz-Halteeinrichtung kann auch mindestens ein Beschleunigungssensor verbunden sein. Mit Hilfe des Beschleunigungssensors können Bewegungen und Aktivitäten des Patienten aufgenommen und bei der späteren Diagnose berücksichtigt werden. Ferner ist es möglich, durch Vorsehen mindestens eines Beschleunigungssensors die Körperlage des Patienten zu bestimmen. Der Beschleunigungssensor ist vorzugsweise über elektrische Leitungen oder drahtlos mit der Aufzeichnungseinrichtung verbunden, so dass die von dem Beschleunigungssensor ermittelten Messdaten wiederum unmittelbar im Bezug zu den EKG-Daten, sowie ggf. im Bezug zu den Blutdruckdaten und den akustischen Aufzeichnungen gesetzt werden können. Vorzugsweise wird ein Beschleunigungssensor im Bereich des Thorax angeordnet. Hierbei kann der Beschleunigungssensor beispielsweise in die Schließeinrichtung integriert sein. Vorzugsweise werden Beschleunigungssensoren mit den Extremitäten des Patienten verbunden. Hierbei werden vorzugsweise mehrere Beschleunigungssensoren vorgesehen, um möglichst exakte Daten über die Aktivitäten des Patienten zu erhalten. Bei dieser Anordnung der Beschleunigungssensoren sind diese vorzugsweise mit drahtlosen Sendeeinrichtungen verbunden, um die Messdaten drahtlos an die Aufzeichnungseinrichtung übermitteln zu können. Damit werden störende elektrische Leitungen vermieden. Der Beschleunigungssensor ist vorzugsweise als 3D bzw. 3 Achs Beschleunigungssensor ausgebildet.

Zur Erfassung der Körperpositionen des Patienten ist auch ein Ultraschallsystem geeignet, das mit kleinen, auf die Körperoberfläche applizierten Ultraschallsensoren arbeitet. Durch genaue Vermessung der Abstände zwischen den Sensoren lassen sich Veränderungen der Körperpositionen erfassen. Die Abstandserfassung erfolgt durch eine Laufzeiterfassung sich oberflächennah im Körpergewebe ausbreitender Ultraschallwellen (Impulsbetrieb). Die Positionserfassung der Wirbelsäule erfolgt dabei vorzugsweise durch seitlich der Wirbelsäule angeordnete Sensoren, die Winkelerfassung von Gliedmaßen durch eine Sensorapplikation im Bereich der jeweiligen Körpergelenke. Die Ultraschallsensoren seitlich der Wirbelsäule können dabei z.T. in das Gurtsystem integriert werden. Bei außerhalb des Gurtsystems aufgeklebten Sensoren werden die Kabel vorzugsweise durch das Gurtsystem geführt. Der erfasste zeitliche Verlauf der Veränderung der Körperpositionen kann ebenfalls vorzugsweise automatisch dem erfassten Verlauf der EKG-Signale zugeordnet werden.

Die Aufzeichnungseinrichtung weist vorzugsweise eine Sende- und/oder Empfangseinrichtung auf. Hierdurch ist es möglich, die aufgezeichneten Daten beispielsweise an eine Auswerteeinrichtung zu übermitteln. Die Sende- und/oder Empfangseinrichtung ist hierbei vorzugsweise derart ausgebildet, dass eine Datenübertragung drahtlos erfolgen kann. Dies hat insbesondere den Vorteil, dass beispielsweise auch eine drahtlose Verbindung der Sende- und/oder Empfangseinrichtung mit einem Mobiltelefon möglich ist. Es ist somit möglich, über das Mobiltelefon ein Notsignal zu versenden, wenn sich der Patient in einem kritischen Zustand befindet, Ein derartiger Zustand kann durch eine mit der Aufzeichnungseinrichtung verbundene Auswerteeinrichtung, die die Daten diesbezüglich auswertet, ermittelt werden.

Die Aufzeichnungseinrichtung ist vorzugsweise mit einem auswechselbaren Akku versehen. Ferner weist die Aufzeichnungseinrichtung zum Speichern der Daten eine vorzugsweise auswechselbare Speichereinrichtung, wie eine SD-Karte auf.

In besonders bevorzugter Ausführungsform ist die Aufzeichnungseinrichtung derart ausgebildet, dass alle erfassten Signale simultan bzw. quasisimultan aufgezeichnet werden. Insbesondere erfolgt die Aufzeichnung zusätzlich in einer korrekten zeitlichen Zuordnung.

Zusätzlich ist es möglich, mit der medizinischen Trageeinrichtung weitere Sensoren insbesondere zur Durchführung einer ambulanten Polysomnographie anzuschließen. Hierzu ist insbesondere ein Schall- bzw. Schwingungsaufnehmer von Lungen-, Atem- und Herzgeräuschen vorgesehen. Diese Aufnehmer müssen ebenfalls anatomisch zuordenbar angeordnet sein. Üblicherweise sind zwei Schallaufnehmer am Rücken dorsal, links und rechts jeweils am sechsten Intercostalraum auf der Medioscapularlinie angeordnet. Ein trachealer Schallaufnehmer ist medial über der Trachea angeordnet. Ferner ist ein Außenschallaufnehmer vorgesehen, der vorzugsweise ebenfalls im Halsbereich nach außen gerichtet ist. Die Sensoren können entweder über gesonderte elektrische Leitungen mit dem Gurtsystem beispielsweise über Steckverbindungen oder andere Kontakte verbunden werden, Ebenso ist es möglich, die einzelnen Gurte des Gurtsystems derart auszugestalten, dass die entsprechenden Sensoren integriert werden können.

Ferner ist es möglich, mit dem Gurtsystem Elektroden zur Ableitung eines EOGs (Elektrookulogramms) und eines EMG (Elektromyogramms) zu verbinden, wobei diese Elektroden üblicherweise in einem Abstand zum Gurtsystem positioniert werden und die Kabel sodann mit dem Gurtsystem verbunden sind.

Des Weiteren können Atemfluss- und Atemdrucksensoren zur Überwachung des nasalen und oralen Atemflusses, sowie Sensoren zur Druckmessung in Verbindung mit einer CPAP-Kontrolle vorgesehen sein.

Auch Dehnungssensoren zur Überwachung von thorakalen und abdominalen Atembewegungen und Atemanstrengungen können vorgesehen werden. Dies ist beispielsweise in der seitlichen Verbindung zwischen Vorder- und Rückteil des Gurtsystems oder im Brustbeingurt möglich.

Ferner kann mit der Trageeinrichtung eine Ortungseinrichtung verbunden sein. Mit Hilfe der Ortungseinrichtung ist es beispielsweise möglich, den Standort des Patienten, beispielsweise über GPS oder GSM oder Galileo, zu ermitteln. Dies kann insbesondere nach dem Absenden eines Notsignals über das Mobiltelefon auch dazu genutzt werden, dass ein Notarzt in kurzer Zeit zum Patienten gelangen kann.

Ferner ist es auch möglich, Lichtsensoren zur Detektion von Licht und Helligkeit vorzusehen.

Nachfolgend wird die Erfindung anhand einer bevorzugten Ausführungsform unter Bezugnahme auf die anliegenden Zeichnungen näher erläutert,

Es zeigen:

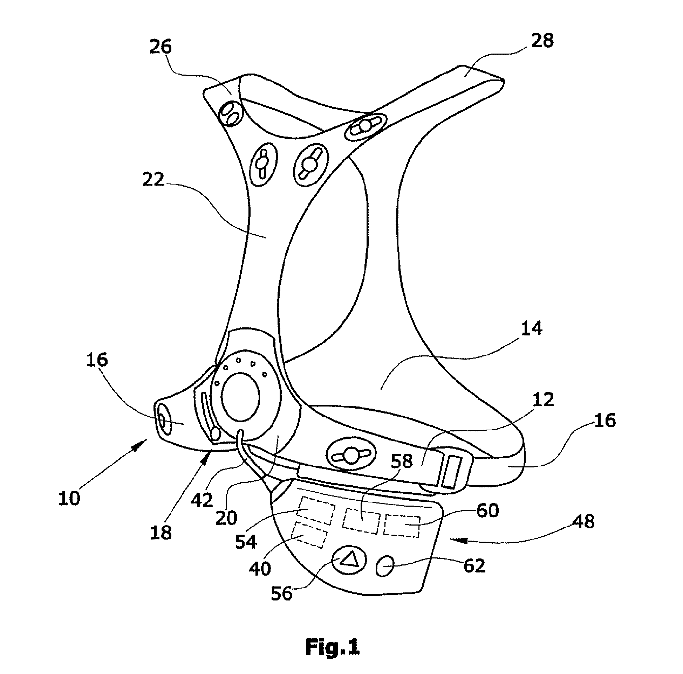

- Fig. 1:: eine schematische, perspektivische Ansicht der medizinischen Trageeinrichtung,
- Fig. 2:: die in Figur 1 dargestellte medizinische Trageeinrichtung, die an einen Patienten angelegt ist, und
- Fig. 3:: eine vergrößerte Ansicht einer Halteeinrichtung.

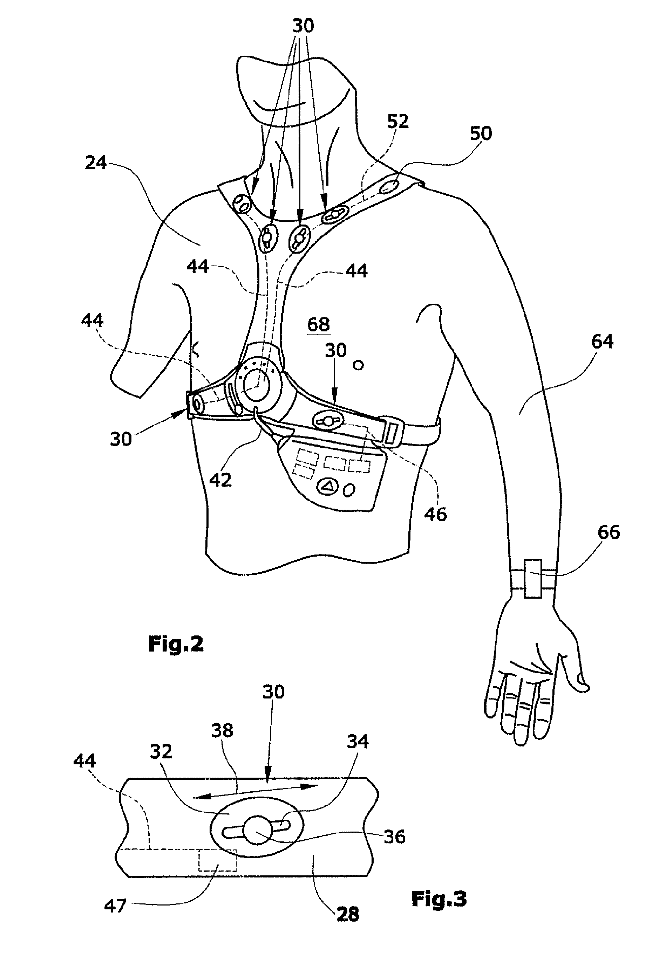

Die medizinische Trageeinrichtung weist ein Gurtsystem auf, das im dargestellten Ausführungsbeispiel einen Brustgurt 10 aufweist. Der Brustgurt 10 weist ein Vorderteil 12 und ein Rückenteil 14 auf. Seitlich ist das Vorderteil auf beiden Seiten mit dem Rückenteil über ein elastisch ausgebildetes Gurtteil 16 zum Ausgleich von Brustkorbbewegungen verbunden. Das Vorderteil 12 ist durch eine Schließeinrichtung 18 unterbrochen, wobei die Schließeinrichtung vorzugsweise im Bereich 20 zu öffnen und zu schließen ist. Ferner ist mit der Schließeinrichtung ein Brustbeingurt 22 vorzugsweise fest verbunden. Der Brustbeingurt 22 liegt an dem Brustbein eines Patienten 24 (Fig. 2) an. Im Bereich der Schlüsselbeine ist der Brustbeingurt 22 mit zwei Schultergurten 26, 28, insbesondere einstückig, verbunden. Die beiden Schultergurte 26, 28 verlaufen am Rücken des Patienten beispielsweise senkrecht nach unten und sind mit dem Rückenteil 14 des Brustgurtes 10 einzeln verbunden. Hierbei kann es sich um eine feste Verbindung handeln. Auch ist es möglich, dass die Verbindung zwischen zumindest einem der Schultergurte 26, 28 und dem Rückenteil 14 des Brustgurtes 10 derart ausgebildet ist, dass ein seitliches Verschieben der Schultergurte zur Anpassung des Gurtsystems an die Anatomie des Patienten möglich ist. Im dargestellten Ausführungsbeispiel sind die Schultergurte am Rücken zusammengeführt.

Mit den Gurten des Gurtsystems sind mehrere Halteeinrichtungen 30 zur Verbindung mit einer EKG-Elektrode verbunden. Die Halteeinrichtungen 30 weisen ein mit der Elektrode verbundenes Halteelement 32 auf. Um eine Justage der Halteeinrichtungen 30 und damit der Elektroden zu ermöglichen, weist das Halteelement 32 eine schlitzförmige Öffnung 34 auf. In der schlitzförmigen Öffnung 34 ist ein stiftförmiger Ansatz 36 vorgesehen. In der schlitzförmigen Öffnung 34 kann der stiftförmige Ansatz 36 zur Justage verschoben werden. Ferner ist das ganze Halteelement 32 dreh- oder schwenkbar in dem Gurt 28 angeordnet. Der stiftförmige Ansatz 36 weist hierbei einen verdickten Kopf auf, so dass der stiftförmige Ansatz 36 zusätzlich zum Halten des Halteelements 32 dient. Auf Grund des Vorsehens der Justageeinrichtung 34, 36 ist ein Verschieben des Halteelements 32 in Richtung des Pfeils 38 möglich.

Eine Stirnseite des stiftförmigen Ansatzes 36, die in Richtung der Patientenoberfläche weist, ist mit einem druckknopfartigem Verbindungselement versehen, um eine Verbindung mit einer handelsüblichen Druckknopf-Elektrode ermöglichen zu können. Ferner sind von der Stirnseite des stiftförmigen Ansatzes ggf. durch diesen in Längsrichtung hindurch Kabel geführt, die sodann in den Gurt 28 integriert sind. Hierbei weisen die nicht dargestellten Kabel beispielsweise eine Schlaufe auf, um ein freies Verschieben des stiftförmigen Ansatzes in dem Schlitz 34 sowie ein Drehen des Halteelements 32 zu ermöglichen.

Ferner ist mit dem Vorderteil 12 des Brustgurtes 10 eine Aufzeichnungseinrichtung 40 verbunden. Die Aufzeichnungseinrichtung 40 ist über elektrische Leitungen 42, 44 mit den Halteeinrichtungen 30 bzw. mit den an den Halteeinrichtungen vorgesehenen Kontakten 47 (Fig. 3) elektrisch verbunden. Hierbei sind die elektrischen Leitungen 44 in die einzelnen Gurte integriert. Die Leitung 42 führt im dargestellten Ausführungsbeispiel zur Schließeinrichtung. Hierzu ist es nicht erforderlich, im Bereich 20, in der die Schließeinrichtung geöffnet und geschlossen werden kann, elektrische Kontakte vorzusehen. Die zu der unmittelbar oberhalb der Aufzeichnungseinrichtung 40 vorgesehenen Halteeinrichtung 30 führende elektrische Leitung 46 kann, wie dargestellt, unmittelbar zur Aufzeichnungseinrichtung führen. Ebenso ist es möglich, die Leitung 44 über das Rückenteil 16 um den Körper des Patienten herum bis zur Schließeinrichtung zu führen und sodann die elektrische Verbindung über das Kabel 42 herzustellen.

Die Aufzeichnungseinrichtung 40 ist vorzugsweise lösbar mit dem Vorderteil 12 des Brustgurtes 10 verbunden, um die Trageeinrichtung möglichst einfach an den Patientenkörper anlegen zu können. Die Aufzeichnungseinrichtung 40 ist in einem Gehäuse 48 angeordnet und weist vorzugsweise ein Speicherelement zum Speichern der Messdaten auf.

Ferner weist die erfindungsgemäße Trageeinrichtung in dem dargestellten Ausführungsbeispiel eine Aufnahmeeinrichtung auf. Diese umfasst ein in dem Schultergurt 28 angeordnetes Mikrophon 50, das über eine elektrische Leitung 52 mit einem Speicherelement 54 verbunden ist. Das Speicherelement 54 ist ebenfalls in dem Gehäuse 48 angeordnet. Ferner umfasst die Aufnahmeeinrichtung einen Aktivierungsknopf 56, der im dargestellten Ausführungsbeispiel an dem Gehäuse 48 vorgesehen ist.

Über eine Zusatz-Halteeinrichtung, bei der es sich um eine der Halteeinrichtungen 30 handeln kann, können weitere medizinische Messgeräte, wie ein Blutdruckmessgerät mit der erfindungsgemäß medizinischen Trageeinrichtung verbunden werden.

In das Gehäuse 48 kann ferner eine Sende- und/oder Empfangseinrichtung 58 integriert sein. Über die Sende- und/oder Empfangseinrichtung 58 kann eine Verbindung mit einem nicht dargestellten Auswertegerät zur Auswertung der medizinischen Daten erfolgen. Ferner kann über die Sende- und /oder Empfangseinrichtung, beispielsweise über Bluetooth, eine Verbindung mit einem Mobiltelefon erfolgen. Hierdurch ist es möglich, ein Notsignal über das Mobiltelefon zu versenden. Die Versendung eines Notsignals kann beispielsweise erfolgen, wenn eine ebenfalls in das Gehäuse 48 integrierte Auswerteeinrichtung 60 auf Grund der gemessenen medizinischen Daten feststellt, dass sich der Patient in einem kritischen Zustand befindet.

Des Weiteren ist es möglich, in das Gehäuse 48 eine Ortungseinrichtung 62 zu integrieren, um einen sich beispielsweise in einem kritischen medizinischen Zustand befindlichen Patienten orten zu können.

Zusätzlich können an Extremitäten des Patienten, wie an einem Arm 64 (Fig. 2) Beschleunigungssensoren 66 vorgesehen sein. Die Beschleunigungssensoren 66 sind drahtlos mit der Sende- und/oder Empfangseinrichtung 58 verbunden, so dass die von den Beschleunigungssensoren 66 ermittelten Daten ebenfalls beispielsweise in der Speichereinrichtung 54 gespeichert werden können. Ein weiterer Beschleunigungssensor kann im Bereich eines Thorax 68 (Fig. 2) des Patienten vorgesehen sein. Insbesondere kann ein Beschleunigungssensor in die Schließeinrichtung 18 integriert sein. Ein in diesem Bereich angeordneter Beschleunigungssensor kann über elektrische Leitungen mit der Speichereinrichtung 54 verbunden sein.

## Patentansprüche

1. Medizinische Trageeinrichtung zum körpernahen Tragen medizinischer Messeinrichtungen, mit
einem Gurtsystem (10, 12, 14, 16, 22, 26, 28),
einer Schließeinrichtung (18) zum Öffnen und Schließen des Gurtsystems (10, 12, 14, 16, 22, 26, 28),
mindestens zwei Halteeinrichtungen (30) zur Aufnahme jeweils einer EKG-Elektrode und
einer mit dem Gurtsystem (10, 12, 14, 16, 22, 26, 28) verbundenen Aufzeichnungseinrichtung (40) zur Aufzeichnung von Messsignalen,

2. Medizinische Trageeinrichtung nach Anspruch 1, **dadurch gekennzeichnet, dass** das Gurtsystem (10, 12, 14, 16, 22, 26, 28) einen Brustgurt (10) umfasst, der vorzugsweise mindestens ein elastisch ausgebildetes Gurtteil (16) zum Ausgleich von Brustkorbbewegungen aufweist.

3. Medizinische Trageeinrichtung nach Anspruch 1 oder 2, **dadurch gekennzeichnet, dass** das Gurtsystem (10, 12, 14, 16, 22, 26, 28) mindestens einen, vorzugsweise zwei Schultergurte (26, 28) aufweist, die vorzugsweise sowohl mit einem Vorderteil (12) als auch einem Rückenteil (14) des Brustgurtes (10) verbunden sind.

4. Medizinische Trageeinrichtung nach Anspruch 3, **dadurch gekennzeichnet, dass** die beiden Schultergurte (26, 28) zur Verbindung mit dem Vorderteil (12) des Brustgurtes (10) mit einem Brustbeingurt (22) verbunden sind.

5. Medizinische Trageeinrichtung nach Anspruch 4, **dadurch gekennzeichnet, dass** der Brustgurt (10) und der Brustbeingurt (22) mit der Schließeinrichtung (18) verbunden sind.

6. Medizinische Trageeinrichtung nach einem der Ansprüche 1 bis 5, **dadurch gekennzeichnet, dass** die Halteeinrichtung (30) ein Justageelement (34, 36) zum Einstellen der Lage der Elektrode aufweist.

7. Medizinische Trageeinrichtung nach Anspruch 6, **dadurch gekennzeichnet, dass** das Justageelement (34, 36) eine schlitzförmige Öffnung (34) aufweist, in der ein stiftförmiger Ansatz (36) verschiebbar gehalten ist.

8. Medizinische Trageeinrichtung nach einem der Ansprüche 1 bis 7, **gekennzeichnet durch** eine vorzugsweise mit dem Gurtsystem (10, 12, 14, 16, 22, 26, 28) verbundene Aufnahmeeinrichtung (50, 54) zur Aufzeichnung von Audiosignalen.

9. Medizinische Trageeinrichtung nach Anspruch 8, **dadurch gekennzeichnet, dass** die Aufnahmeeinrichtung ein vorzugsweise in den Schultergurt (28) integriertes Mikrophon (50) aufweist.

10. Medizinische Trageeinrichtung nach einem der Ansprüche 1 bis 9, **gekennzeichnet durch** eine mit dem Gurtsystem (10, 12, 14, 16, 22, 26, 28) verbundene Zusatz-Halteeinrichtung (30) zum Verbinden mit medizinischen Sensoren oder Modulen.

11. Medizinische Trageeinrichtung nach einem der Ansprüche 1 bis 10, **gekennzeichnet durch** an den Halteeinrichtungen (30) vorgesehenen elektrischen Kontakten (47).

12. Medizinische Trageeinrichtung nach einem der Ansprüche 1 bis 11, **gekennzeichnet durch** zwischen den Halteeinrichtungen (30) und der Aufzeichnungseinrichtung (40) angeordneten, insbesondere in die Gurte integrierten elektrischen Leitungen (42, 44).

13. Medizinische Trageeinrichtung nach einem der Ansprüche 1 bis 12, **dadurch gekennzeichnet, dass** die Schließeinrichtung (18) elektrische Kontakte aufweist.

14. Medizinische Trageeinrichtung nach einem der Ansprüche 1 bis 13, **dadurch gekennzeichnet, dass** die Aufzeichnungseinrichtung (40) mit einer Sende- und/oder Empfangseinrichtung (58) verbunden ist.

15. Medizinische Trageeinrichtung nach einem der Ansprüche 1 bis 14, **gekennzeichnet durch** eine Ortungseinrichtung (62), die vorzugsweise mit der Sende- und/oder Empfangsseinrichtung (58) verbunden ist.

16. Medizinische Trageeinrichtung nach einem der Ansprüche 1 bis 15, **gekennzeichnet durch** mindestens einen Beschleunigungssensor, der vorzugsweise mit dem Gurtsystem (10, 12, 14, 16, 22, 26, 28) insbesondere im Bereich des Thorax (68) verbunden ist.

17. Medizinische Trageeinrichtung nach Anspruch 16, **dadurch gekennzeichnet, dass** der Beschleunigungssensor (66) mit Extremitäten (64) des Patienten (24) verbunden ist und eine Sendeeinrichtung zum drahtlosen Verbinden mit der Aufzeichnungseinrichtung (40) aufweist.

18. Medizinische Trageeinrichtung nach einem der Ansprüche 1 bis 17, **gekennzeichnet durch** eine Positionserfassungsvorrichtung zur Erfassung der Körperpositionen des Patienten, wobei die Positionserfassungsvorrichtung, vorzugsweise Ultraschallsensoren, aufweist.

19. Medizinische Trageeinrichtung nach Anspruch 18, **gekennzeichnet durch** eine Laufzeiterfassungsvorrichtung zur Messung des Abstandes der Ultraschallsensoren an verschiedenen Körperteilen des Patienten.

20. Verfahren zur Erfassung eines Langzeit-EKG's, insbesondere unter Verwendung einer Vorrichtung nach einem der Ansprüche 1 bis 19 mit den Schritten
- Aufzeichnen von EKG-Signalen durch eine Aufzeichnungseinrichtung (40)
- Aufnahme von Audiosignalen durch eine Aufnahmevorrichtung (50, 54) und
- automatisiertes Zuordnen der EKG-Signale zu den entsprechenden Audiosignalen.

21. Verfahren nach Anspruch 20, **gekennzeichnet durch** den zusätzlichen Schritt:
- Erfassen einer Beschleunigung von Körperteilen des Patienten, **durch** mindestens einen Beschleunigungssensor.

22. Verfahren nach Anspruch 21, **gekennzeichnet durch** den zusätzlichen Schritt:
- Zuordnen des zeitlichen Verlaufs der erfassten Beschleunigung zum erfassten zeitlichen Verlauf der EKG-Signale,

23. Verfahren nasch einem der Ansprüche 20 bis 22, **gekennzeichnet durch** den zusätzlichen Schritt:
- Erfassen einer Bewegung mindestens eines Körperteils des Patienten **durch** einen Positionserfassungssensor, der insbesondere als Ultraschallsensor ausgebildet ist.

24. Verfahren nach Anspruch 23, **gekennzeichnet durch** den zusätzlichen Schritt:
- Zuordnen des erfassten zeitlichen Verlaufs der Bewegung eines Körperteils des Patienten zum erfassten zeitlichen Verlauf der EKG-Signale.

25. Verfahren nach einem der Ansprüche 20 bis 24 mit dem zusätzlichen Schritt:
- Aufzeichnen eines Zeitsignals, das den EKG- und Audiosignalen zugeordnet wird.
